# EUROPÄISCHE PATENTSCHRIFT

(11) **EP 2 506 858 B1**
(45) Veröffentlichungstag und Bekanntmachung des Hinweises auf die Patenterteilung: **10.05.2017**
(21) Anmeldenummer: 10790392.4
(22) Anmeldetag: 06.12.2010
(51) Int. Cl.: A61K 31/718, A61K 47/36, C08B 31/10, C08B 33/00, A61K 9/00, A61P 9/08, A61P 9/14

(54) **STÄRKEDERIVATMISCHUNGEN**
MIXTURES OF STARCH DERIVATIVES
MELANGES DE DERIVES DE L'AMIDON

(30) Priorität: 06.12.2009 DE 102009056832
(43) Veröffentlichungstag der Anmeldung: 10.10.2012
(73) Patentinhaber: B. Braun Melsungen AG, 34212 Melsungen (DE)
(72) Erfinder: MEIER, Bernd, 64285 Darmstadt (DE); JANKOWIAK-MEIER, Iris, Theresia, 64285 Darmstadt (DE); MEIER, Nele, 64285 Darmstadt (DE); MEIER, Clara, 64285 Darmstadt (DE)
(74) Vertreter: dompatent von Kreisler Selting Werner - Partnerschaft von Patent- und Rechtsanwälten mbB
(86) Internationale Anmeldenummer: PCT/EP2010/068950
(87) Internationale Veröffentlichungsnummer: WO 2011/067403

(56) Entgegenhaltungen:
- EP-A1- 1 190 719
- EP-A1- 1 473 308
- EP-A2- 1 230 935
- WO-A2-2005/082942
- DE-A1- 4 123 001

## Beschreibung

Die Erfindung betrifft Zusammensetzungen umfassend ein erstes Stärkederivat und ein zweites Stärkederivat, wobei das mittlere Molekulargewicht des ersten Stärkederivats größer ist als das mittlere Molekulargewicht des zweiten Stärkederivats und der Substitutionsgrad des zweiten Stärkederivats größer ist als der Substitutionsgrad des ersten Stärkederivats. Weiterhin betrifft die Erfindung eine pharmazeutische Zubereitung, die eine solche Stärkederivatzusammensetzung umfasst sowie ein Verfahren zur Herstellung einer solchen Zusammensetzung.

Das Dilemma künstlicher Plasmaersatzlösungen besteht darin, dass die chemische Modifikation von Biopolymeren einerseits in Blut lösliche und stabile Kolloide bereitstellt, andererseits jedoch Kolloidmoleküle im Körper belässt, die in den Organen gespeichert werden. Die Infusion von Hydroxyalkylstärke- und Carboxyalkylstärke-Lösungen, dient dem Ersatz von Blut bzw. dessen Plasmabestandteilen. Durch die infundierten Kolloide soll der physiologische kolloidosmotische Druck, der im Wesentlichen auf Albumin zurückgeführt wird, aufrecht erhalten werden. Die durch die dispergierten Kolloide bewirkte Dampfdruckerniedrigung, fördert den Einstrom von Wasser aus dem interstitiellen Gewebe in die Blutgefäße. Diese Wirkung spielt insbesondere bei septischen Patienten, bei denen es durch Schrankenstörungen zwischen interstitiellem Gewebe und Blut zu erheblichen Verschiebungen von Wasser aus dem Blut in das interstitielle Gewebe kommt, eine wesentliche Rolle. Weitere Wirkungen sind, der positive Effekt des in den Blutgefäßen gehaltenen Wassers auf die Fließeigenschaften des Blutes sowie die Begünstigung von laminaren Strömungen in den Gefäßen. Hydroxyalkylstärken werden in der Regel aus Kartoffel- oder Wachsmaisstärke hergestellt. Als natürlich gewachsene Polymerverbindungen weisen sie von Molekül zu Molekül sehr unterschiedliche Molekulargewichte und Verzweigungen auf. Nach hydrolytischer Spaltung zu kleineren einheitlicheren Molekülen, die relativ eng um einen definierten Mittelwert des Molekulargewichts verteilt sind, wird eine definierte Menge von Hydroxyalkylgruppen zur Verbesserung der Wasserlöslichkeit und zum Schutz vor der enzymatischen Spaltung in das Kolloid eingeführt. Dergleichen modifizierte Stärkepräparate werden durch zwei Größen, den molaren Substitutionsgrad MS und das mittlere Molekulargewicht Mw charakterisiert. Der molare Substitutionsgrad MS entspricht der Gesamtzahl der Hydroxyethylgruppen pro Gesamtzahl der Glucosemoleküle. Das Molekulargewicht Mw entspricht dem Massenmittel aller Moleküle des polydispersen Kolloidgemisches. Die enge Molekulargewichtsverteilung um das mittlere Molekulargewicht gilt dabei als ein Gütekriterium für die als Arzneimittel zugelassenen Hydroxyethylstärkelösungen. Die Elimination von Hydroxy- und Carboxyalkylstärke wird in erster Linie vom molaren Substitutionsgrad MS abhängig gemacht. Der Einfluss des molaren Substitutionsgrades auf die Eliminationsgeschwindigkeit für Hydroxyethylstärke beruht auf der sterischen Behinderung des enzymatischen Angriffs hydrolytisch wirkender Enzyme. Je höher die Anzahl der in das Stärkemolekül eingeführten Hydroxyethylgruppen, umso langsamer wird die Hydroxyethylstärke ausgeschieden. Der Infusion von Hydroxyalkylstärken sind aufgrund der Speicherung im RHS (Retikulohistiozytäres System) und den daraus resultierenden sehr kontrovers diskutierten Beeinträchtigungen, quantitative Grenzen gesetzt. Da vor allem hoch substituierte Kolloide stärker phagozytiert und gespeichert werden, ist der molare Substitutionsgrad neuerer HES-Lösungen deutlich abgesenkt. Der hydrolytische Abbau der Hydroxyethylstärke wird im Blut vor allem mit der Aktivität der alpha-Amylase erklärt. Das Enzym, ein Tetramer mit einem Molekulargewicht von ca. 60 kDa, spaltet von den endständigen Teilen der Hydroxyethylstärke Glucosereste, Maltose bzw. Maltotriosen ab. Die Spaltung der 1,6-Verzweigungsstellen und hydrolytische Abtrennung längerer Reste sind in Abhängigkeit vom Verzweigungsgrad der Ausgangsstärke seltener. Nach Infusion steigt der Anteil der an C2-substituierten Hydroxyalkylglucosen gegenüber an den C6-substitutierten Molekülen deutlich an. Bei Hydroxyethylstärkeinfusionen bei gesunden Probanden wurden von Weitler und Sommermeier und Förster et al. folgende Beobachtungen gemacht:

Die Breite der im Blut vorliegenden Molekulargewi-chtsverteilung der Hydroxyethylstärke nimmt nach Infusion mit der Zeit ab. Im Urin nimmt das mittlere Molekulargewicht mit der Zeit deutlich zu. Nach Infusion einer Hydroxyethylstärke (HES) (z.B. Mw:200 kDa; MS:0,5) steigt das Molekulargewicht der im Urin ausgeschiedenen HES an (von 20 kDa auf 40 kDa). Im Urin gesunder Probanden werden keine Molekulargewichte über 70 kDa gefunden. Die Substitution der im Urin ausgeschiedenen HES nimmt vergleichbar zu, wie die molare Substitution der im Serum verbliebenen Hydroxyethylstärke zunimmt.

Es wird angenommen, dass diese Moleküle als sogenannte Reststärken von den Zellen des RHS gespeichert werden. Während nun die Serumhalbwertszeit einer HES hinreichend mit ihrem Substitutionsgrad korreliert, was die sterische Behinderung hydrolytischer Enzyme, insbesondere der Serumamylase durch die eingeführte Hydroxyalkylgruppen nahelegt, bleibt der Verlauf der ausgeschiedenen Molekulargewichtsgrößen im Urin ungeklärt.

Der Ersatz intravasaler Flüssigkeit gehört zu den wichtigsten Maßnahmen bei der Prophylaxe und Therapie der Hypovolämie und zwar unabhängig davon, ob die Hypovolämie aus dem unmittelbaren Verlust von Blut bzw. Körperflüssigkeiten resultiert (bei akuten Blutungen, Traumen, Operationen, Verbrennungen), aus Verteilungsstörungen zwischen Makro- und Mikrozirkulation (wie bei Sepsis) oder aus einer Vasodilation (z. B. bei der Anästhesieeinleitung). Für diese Indikationen geeignete Infusionslösungen sollen die Normovolämie wieder herstellen und die Perfusion lebenswichtiger Organe sowie den peripheren Blutfluss aufrechterhalten. Gleichzeitig dürfen die Lösungen den Kreislauf nicht übermäßig belasten und sie müssen von Nebenwirkungen möglichst frei sein. In dieser Hinsicht bieten sämtliche derzeit verfügbaren Volumenersatzlösungen Vor- und Nachteile. Sogenannte kristalloide Lösungen (Elektrolytlösungen) sind zwar weitgehend frei von unmittelbaren Nebenwirkungen, gewährleisten aber nur kurzfristige oder inadäquate Stabilisierung des intravasalen Volumens und der Hämodynamik. Bei ausgeprägter oder länger anhaltender Hypovolämie müssen sie in exzessiven Mengen infundiert werden, da sie nicht ausschließlich im intravasalen Kompartiment verbleiben, sondern sich rasch im extravasalen Raum verteilen. Ein schneller Abstrom in den extravasalen Raum limitiert aber nicht nur die kreislauffüllende Wirkung kristalloider Lösungen, sondern birgt auch das Risiko peripherer und pulmonaler Ödeme. Abgesehen von der vitalen Bedrohung, die ein Lungenödem darstellen kann, führt es außerdem zur Verschlechterung der nutritiven Sauerstoffversorgung, die durch periphere Ödeme ebenfalls beeinträchtigt wird.

Demgegenüber sind kolloidale Volumenersatzlösungen, seien die in ihnen enthaltenen Kolloide natürlichen oder synthetischen Ursprungs, in ihrer Wirkung weitaus zuverlässiger. Dies ist darauf zurückzuführen, dass sie aufgrund ihrer kolloidosmotischen Wirkung die zugeführte Flüssigkeit länger im Kreislauf zurückhalten als Kristalloide und sie so vor dem Abstrom in das Interstitium schützen. Andererseits geben kolloidale Volumenersatzlösungen in höherem Maß als kristalloide Lösungen zu unerwünschten Reaktionen Anlass. So birgt das natürliche Kolloid Albumin, wie alle Blut- bzw. Plasmaderivate, das Risiko der Ansteckung mit viralen Erkrankungen; zusätzlich kann es zu Wechselwirkungen mit anderen Arzneimitteln, z. B. ACE-Inhibitoren, kommen; schließlich ist die Verfügbarkeit von Albumin limitiert und seine Verwendung als Volumenersatzmittel unverhältnismäßig teuer. Weitere Bedenken gegen den Einsatz von Albumin als Volumenersatzmittel haben ihren Grund in der Hemmung der endogenen Synthese von Albumin bei dessen exogener Zufuhr und in seiner leichten Extravasation. Hierunter wird der Übertritt aus dem Kreislauf in den extravasalen Raum verstanden, wo es wegen des kolloidosmotischen Effekts von Albumin zu unerwünschten und langanhaltenden Flüssigkeitsansammlungen kommen kann.

Bei den synthetischen Kolloiden haben schwere anaphylaktoide Reaktionen und eine massive Beeinträchtigung der Blutgerinnung dazu geführt, dass Dextran-Präparate aus der Therapie nahezu völlig verschwunden sind. Hydroxyethylstärke (HES)-Lösungen besitzen zwar ebenfalls das Potential zur Auslösung anaphylaktoider Reaktionen und zur Beeinflussung der Blutgerinnung, dies jedoch in geringerem Maß als Dextran. Schwerwiegende anaphylaktoide Reaktionen (Reaktionen der Schweregrade III und IV) werden mit HES-Lösungen - anders als mit Dextran - äußerst selten beobachtet; und der Einfluss auf die Blutgerinnung, der den hochmolekularen HES-Lösungen eigen ist, konnte durch die Weiterentwicklung von HES-Lösungen in den letzten Jahren erheblich vermindert werden. Im Vergleich mit den Gelatinelösungen, die ebenfalls als Plasmaersatzmittel Verwendung finden und die Blutgerinnung weitgehend unbeeinflusst lassen, besitzen HES-Lösungen, zumindest deren hoch- und mittelmolekulare Ausführungsformen, den Vorzug längerer Plasmaverweildauer und Wirksamkeit.

EP-A-0402724 offenbart die Herstellung und Verwendung einer Hydroxyethylstärke mit einem mittleren Molekulargewicht Mw von 60000 bis 600000, einem molaren Substitutionsgrad MS von 0,15 bis 0,5 und einem Substitutionsgrad DS von 0,15 bis 0,5. Die Offenbarung beschäftigt sich mit der schnellen (6 bis 12 Stunden) und vollständigen Abbaubarkeit der als Plasmaexpander einzusetzenden Hydroxyethylstärken. Explizit untersucht wurde dabei innerhalb des bevorzugten mittleren Molekulargewichtsbereichs von 100000 bis 300000 eine Hydroxyethylstärke mit einem mittleren Molekulargewicht von 234000.

US-A-5,502,043 offenbart die Verwendung von Hydroxyethylstärken mit einem mittleren Molekulargewicht Mw von 110000 bis 150000, einer molaren Substitution MS von 0,38 bis 0,5 und einem Substitutionsgrad DS von 0,32 bis 0,45 zur Verbesserung der Mikrozirkulation bei peripherer arterieller Durchblutungsstörung. Darüber hinaus lehrt die Druckschrift den Einsatz von niedermolekularen (Mw 110000 bis 150000) Hydroxyethylstärken, die bedingt durch ihr niedriges Molekulargewicht die Plasmaviskosität niedrig halten und somit eine Verbesserung der Mikrozirkulation im Blutkreislauf gewährleisten. Abgeraten wird in dieser Schrift jedoch von dem Einsatz höhermolekularer Hydroxyethylstärken, wie etwa einer Hydroxyethylstärke mit einem Mw: 500000, da diese trotz niedriger molarer Substitution (MS=0,28) die Plasmaviskosität erhöhen und somit die Mikrozirkulation verschlechtern.

WO 2005/082942 A2 offenbart ein Verfahren zur Herstellung einer Hydroxyethylstärke durch Umsetzung von in Wasser suspendierter Stärke mit Ethlyenoxid und anschließender Teilhydrolisierung des erhaltenen Stärkederivats mit Säure bis zum gewünschten mittleren Molekulargewichtsbereich der Hydroxyethylstärke.

Weltweit werden zur Zeit unterschiedliche HES-Präparate als kolloidale Volumenersatzmittel verwendet, die sich hauptsächlich in ihrem Molekulargewicht und daneben in dem Ausmaß der Veretherung mit Hydroxyethylgruppen und in anderen Parametern unterscheiden. Die bekanntesten Vertreter dieser Substanzklasse sind die sog. Hetastarch (HES 450/0,7) und Pentastarch (HES 200/0,5). Bei letztgenannter handelt es sich um die derzeit am weitesten verbreitete "Standard-HES". Neben dieser spielen HES 200/0,62 und HES 70/0,5 eine geringere Rolle. Bei den deklarierten Angaben zum Molekulargewicht handelt es sich ebenso wie bei denjenigen zu den anderen Parametern um gemittelte Größen, wobei der Molekulargewichtsdeklaration das Massenmittel (Mw) zugrunde liegt, das in Dalton (z. B. bei HES 200000) oder meist verkürzt in Kilodalton (z. B. wie bei HES 200) angegeben wird. Das Ausmaß der Veretherung mit Hydroxyethylgruppen wird durch die molare Substition MS (z.B. als 0,5 wie in HES 200/0,5; MS = durchschnittliches molares Verhältnis von Hydroxyethylgruppen zu Anhydroglucoseeinheiten) oder durch den Substitutionsgrad (DS = Verhältnis von ein- oder mehrfach hydroxyethylierten Glucosen zu den Gesamt-Änhydroglucoseeinheiten) gekennzeichnet. Entsprechend ihrem Molekulargewicht werden die in klinischem Gebrauch befindlichen HES-Lösungen in hochmolekulare (450 kD), mittelmolekulare (200 - 250 kD) und niedermolekulare Präparate (70 - 130 kD) eingeteilt.

Was die Gerinnungseffekte von HES-Lösungen angeht, so ist zwischen unspezifischen und spezifischen Einflüssen zu unterscheiden. Zu einer unspezifischen Beeinflussung der Blutgerinnung kommt es aufgrund der Verdünnung des Bluts (Hämodilution), die bei der Infusion von HES- und anderen Volumenersatzlösungen in den Blutkreislauf stattfindet. Von dieser Hämodilution sind auch Gerinnungsfaktoren betroffen, deren Konzentrationen je nach Ausmaß und Dauer der infusionsbedingten Verdünnung des Bluts und der Plasmaproteine absinken. Entsprechend große oder anhaltende Effekte können zu einer labordiagnostisch nachweisbaren, in extremen Fällen zu einer klinisch relevanten Hypokoagulabilität führen.

Daneben kann von der Hydroxyethylstärke eine spezifische Beeinflussung der Blutgerinnung ausgehen, für die mehrere Faktoren verantwortlich gemacht werden. So findet man - unter bestimmten Bedingungen bzw. mit bestimmten HES-Präparaten - einen Abfall der Gerinnungsproteine Faktor VIII (F VIII) und von Willebrand-Faktor (vWF), der größer ist als die hämodilutionsbedingte Abnahme der Plasmaproteine im Allgemeinen. Ob dieser stärkere als zu erwartende Abfall durch eine verminderte Bildung bzw. Freisetzung von F VIII/vWF bedingt ist, etwa durch HES-bedingte Coating-Effekte am Gefäßendothel oder durch andere Mechanismen, ist nicht völlig geklärt.

HES beeinflusst aber nicht nur die Konzentration der genannten Gerinnungsfaktoren, sondern offenbar auch die Funktion der Blutplättchen. Hierfür ist ganz oder teilweise die Bindung von HES an die Oberfläche der Blutplättchen verantwortlich, die den Zutritt von Liganden an den Fibrinogenrezeptor der Plättchen hemmt.

Diese spezifischen Wirkungen von HES auf die Blutgerinnung sind bei der Anwendung hochmolekularer HES (z. B. von HES 450/0,7) besonders ausgeprägt, während sie bei mittelmolekularer (z. B. HES 250/0,5) oder niedermolekularer HES (z. B. HES 130/0,4 oder HES 70/0,5) keine so große Rolle mehr spielen (J. Treib et al., Intensive Care Med. (1999), S. 258 bis 268; O. Langeron et al., Anesth. Analg. (2001), S. 855 bis 862; R.G. Strauss et al., Transfusion (1988), S. 257 - 260; M. Jamnicki et al., Anesthesiology (2000), S. 1231 bis 1237).

Vergleicht man das Risikoprofil hochmolekularer HES mit dem der mittel- und niedermolekularen Präparate, so kann man bei den letztgenannten eine deutliche Reduzierung der Risiken feststellen, und zwar nicht nur im Hinblick auf die Wechselwirkung mit der Blutgerinnung, sondern auch im Hinblick auf bestimmte pharmakokinetische Eigenschaften. So zeigen die hochmolekularen HES-Lösungen eine starke Akkumulation im Kreislauf, während dieser Nachteil bei mittelmolekularer HES nur in abgeschwächter Form und bei niedermolekularen Präparaten praktisch nicht mehr vorhanden ist. Dass mit niedermolekularen HES-Lösungen wie mit HES 130/0,4 keine Kumulation mehr auftritt, ist ein relevanter therapeutischer Fortschritt, weil sich die Plasmakonzentrationen von HES in der klinischen Routine nicht bestimmen lassen und selbst extreme Konzentrationen, zu denen es mit den hochmolekularen Lösungen binnen weniger Tage kommen kann, daher unentdeckt bleiben. Bei diesen ist die Menge der im Kreislauf kumulierenden "Rest-HES" dem Anwender unbekannt, sie beeinflusst aber gleichwohl die Kinetik und das Verhalten derjenigen HES, die in Unkenntnis der im Kreislauf noch vorhandenen Mengen additiv infundiert wurde. Hochmolekulare HES entsprechend dem Stand der Technik ist in ihrer Wirkung daher nicht berechenbar; sie bleibt länger im Kreislauf als dies aus therapeutischen Gründen in den meisten Fällen erforderlich oder erwünscht wäre und ihr metabolisches Schicksal ist ungeklärt.

Im Unterschied dazu verschwindet niedermolekulare HES innerhalb von etwa 20 bis 24 Stunden nach der Infusion vollständig aus dem Kreislauf. Dadurch werden Überhangeffekte vermieden und es kommt - speziell bei wiederholter Infusion - nicht zur Akkumulation. Das pharmakokinetische Verhalten von niedermolekularer Stärke ist - anders als bei hochmolekularer Stärke - berechenbar und lässt sich daher leicht steuern. Eine zu starke Belastung des Kreislaufs oder der Eliminationsmechanismen tritt nicht auf.

Dieses - für sich genommen - vorteilhafte Verhalten niedermolekularer HES im Vergleich mit hochmolekularen Präparaten wird jedoch mit einer erheblich kürzeren Plasmahalbwertszeit erkauft. Die Plasmahalbwertszeit von niedermolekularer HES beträgt nur etwa die Hälfte derjenigen von HES 200 oder weniger (J. Waitzinger et al., Clin. Drug Invest. (1998) S. 151 bis 160) und liegt im Bereich der Halbwertszeit von Gelatinepräparaten, die als ausgesprochen kurzwirksam einzustufen sind. Zwar muss eine kurze Halbwertszeit eines Volumenersatzmittels nicht grundsätzlich von Nachteil sein, da sie durch öftere bzw. höher dosierte Verabreichung des betreffenden Volumenersatzmittels kompensiert werden kann. Bei schwerer oder anhaltender Hypovolämie birgt ein Volumenersatzmittel mit kurzer Halbwertszeit und kurzer Wirkdauer jedoch die Gefahr unzureichender Kreislauffüllung (ähnlich wie mit kristalloiden Lösungen) bzw. - bei entsprechend höherer Dosierung zur Kompensation dieses Nachteils - das Risiko interstitieller Flüssigkeitsüberladung.

Vor diesem Hintergrund besteht ein Bedarf an einem Volumenersatzmittel, das sich einerseits durch geringe Kumulationsneigung und geringe Beeinflussung der Blutgerinnung auszeichnet (wie niedermolekulare HES), andererseits aber eine längere Halbwertszeit aufweist als die niedermolekularen, den kristalloiden Lösungen nahestehenden HES-Lösungen.

Es wurde nun überraschend gefunden, dass Zusammensetzungen umfassend mindestens zwei unterschiedliche Stärkederivate, die im Stand der Technik genannten Probleme lösen und insbesondere eine längere Plasmahalbwertszeit und gleichzeitig eine Verringerung der verbleibenden Reststärke erzielt werden kann.

Gegenstand der Erfindung ist daher eine wässrige pharmazeutische Zubereitung wie in Anspruch 1 definiert. Bevorzugte Ausgestaltungen der Zubereitung gehen aus den abhängigen Ansprüchen hervor.

Die erfindungsgemäße Zusammensetzung ist bevorzugt auf eine minimale Organspeicherung optimiert, wobei das erste und das zweite Stärkederivat ein hinreichend unterschiedliches mittleres Molekulargewicht aufweisen und dergestalt zusammengesetzt sind, dass die Stärkederivate mit höherem Molekulargewicht niedriger und die Stärkederivate mit niedrigerem Molekulargewicht höher substituiert sind.

Der molare Substitutionsgrad MS1 der Stärkederivate mit größerem Molekulargewicht ist sehr niedrig gewählt, wodurch eine Spaltung in kleinere Molekulargewichtsgrößen möglich ist und eine Speicherung in den Organen weitgehend unterbleibt. Die zweiten Stärkederivate, welche niederigere mittlere Molekulargewichte aufweisen, vorzugsweise hinreichend nahe an der Ausschlußgrenze der Niere, werden im Gegenzug entsprechend höher substituiert und weisen entsprechend einen hohen MS2-Wert auf.

Gegenüber herkömmlichen Hydroxy-alkyl-stärkelösungen deren Molekulargewichtsfraktionen in einem Prozess gemeinsam hydroxyethyliert werden, sind die erfindungsgemäßen Zusammensetzungen asymmetrisch substituiert. Die molare Substitution MS nimmt mit steigendem Molekulargewichts stark ab. Dabei werden kleinere Moleküle durch die höhere Substitution mit größeren hydrodynamischen Radien versehen als die größeren Moleküle in der Lösung.

In einer bevorzugten Ausführungsform umfasst die erfindungsgemäße Zusammensetzung mindestens 2 Stärkederivate, vorzugsweise Hydroxyethylstärke und/oder Acetylstärke, wobei das zweite Stärkederivate mit niedrigerem mittleren Molekulargewicht im Vergleich zum ersten Stärkederivat, deutlich höher und das erste Stärkederivat (mit größerem mittleren Molekulargewicht) deutlich niedriger substituiert sind. In vorteilhafter Ausgestaltung ist eine HES oder eine Acetylstärke mit einem MW im Bereich der Nierenschwelle so hoch substituiert, dass sie vor dem Angriff der Serumamylase weitgehend geschützt ist, jedoch unter Berücksichtigung ihrer durch die Substitution vermehrten hydrodynamischen Radien noch gut renal eliminiert werden kann. Das erste Stärkederivat (mit größerem Molekulargewicht) ist im Gegenzug sehr viel niedriger substituiert. In einer weiteren vorteilhaften Ausgestaltung wird die Größenverteilung den physiologischen Verhältnissen des menschlichen Blutes angepasst. Hierzu liegt das mittlere Molekulargewicht des zweiten Stärkederivat in der Größenordnung des Albumins, und das mittlere Molekulargewicht des ersten Stärkederivats im Bereich der Globuline.

Die erfindungsgemäß einzusetzenden Stärkederivate werden durch den molaren Substitutionsgrad MS beeinflusst. Der molare Substitutionsgrad MS (molar substitution) ist definiert als die durchschnittliche Anzahl von Hydroxyethylgruppen pro Anhydroglucose-Einheit (Sommermeyer et al., Krankenhauspharmazie (1987), S. 271 bis 278). Der molare Substitutionsgrad kann gemäss Ying-Che Lee et al. Anal. Chem. (1983) 55, 334 und K.L. Hodges et al., Anal. Chem (1979) 51, 2171 bestimmt werden. Eine bekannte Menge des Stärkederivats, beispielsweise einer Hydroxyethylstärke (HES), wird dabei unter Zusatz von Adipinsäure und Iodsäure (HI) in Xylol einer Etherspaltung unterzogen. Das freigesetzte Ethyliodid wird anschliessend gaschromatographisch unter Verwenden eines internen Standards (Toluol) und externer Standards (Ethyliodid-Eichlösungen) quantifiziert. Der molare Substitutionsgrad MS beeinflusst die Wirkung der erfindungsgemäß einzusetzenden Stärkederivate.

Die erfindungsgemäß einzusetzenden Stärkederivate liegen herstellungsbedingt nicht als Molekular-einheitliche Substanzen mit definiertem Molekulargewicht vor, sondern als Gemisch von Molekülen unterschiedlicher Größe, die auch verschieden substituiert sein können. Die Charakterisierung solcher Gemische bedarf daher der Zuhilfenahme statistisch gemittelter Größen. Zur Kennzeichnung des durchschnittlichen Molekulargewichts dient daher das gemittelte Molekulargewicht (Mw), wobei die allgemeine Definition dieses Mittelwertes in Sommermeyer et al., Krankenhauspharmazie (1987), S. 271 bis 278 angegeben ist.

Die Molekulargewichtsbestimmung kann mittels GPC-MALLS unter Verwendung der GPC-Säulen TSKgel G 6000 PW, G 5000 PW, G 3000 PW und G 2000 PW (7,5 mm x 30 cm), des MALLS-Detektors (DAWN-EOS; Wyatt Deutschland GmbH, Woldert) und des RI-Detektors (Optilab DSP; Wyatt Deutschland GmbH, Woldert) bei einer Flussrate von 1,0 ml/Minute in einem 50 mM Phosphatpuffer pH 7,0 erfolgen. Die Auswertung kann mittels ASTRA-Software (Wyatt Deutschland GmbH, Woldert) durchgeführt werden.

Bevorzugt sind solche Stärkederivate, die aus nativen oder partiell hydrolysierten Getreide- oder Kartoffelstärken erhältlich sind. Insbesondere bevorzugt sind hierbei Wachs-Varietäten, der entsprechenden Pflanzen, sofern diese existieren (z.B. Wachsmais oder Wachsreis).

Es hat sich überraschend gezeigt, dass Stärkederivate, die aus Stärken hergestellt wurden, die überwiegend aus Amylose bestehen, besonders geeignet sind im Rahmen der vorliegenden Erfindung. Gerade aus solchen Stärkederivaten hergestellte erfindungsgemäße Zusammensetzungen sind geeignet für wässrige Infusionslösungen, deren Viskosität sich in einem ungewöhnlich starken Ausmaß über die Zugabe von Elektrolyten einstellen lassen.

In einer besonders bevorzugten Ausführungsform der vorliegenden Erfindung, basieren die erfindungsgemäß einzusetzenden Stärkederivate auf Stärken, die zumindest 50 Gew.-%, vorzugsweise mindestens 65 Gew.-% und insbesondere mindestens 75 Gew.-% Amylose aufweisen, wobei das Gewicht auf das Gesamtgewicht der Stärke bezogen ist.

Die erfindungsgemäß einzusetzenden Stärkederivate können ferner durch das Verhältnis der Substitution an C₂ zur Substitution an C₆ der Anhydroglucose-Einheiten beschrieben werden. Dieses Verhältnis, das im Rahmen dieser Erfindung auch als C₂/C₆-Verhältnis abgekürzt wird, bedeutet das Verhältnis der Zahl der in 2-Stellung substituierten Anhydroglucose-Einheiten zu der Zahl der in 6-Stellung substituierten Anhydroglucose-Einheiten der Stärkederivate. Das C₂/C₆-Verhältnis einer HES kann beispielsweise durch die Menge der bei der Hydroxyethylierung verwendeten Natronlauge in weiten Grenzen variiert werden. Je höher die eingesetzte Menge an NaOH ist, umso stärker werden die Hydroxygruppen in 6-Stellung bei der Anhydroglucose der Stärke für die Hydroxyethylierung aktiviert. Daher nimmt das C₂/C₆-Verhältnis mit steigender NaOH-Konzentration während der Hydroxyethylierung ab. Die Bestimmung erfolgt wie von Sommermeyer et al., Krankenhauspharmazie (1987), S. 271 bis 278, angegeben. Die C_{2/}C₆-Verhältnisse für das erste und/oder das zweite Stärkederivat betragen in der nachfolgenden Reihenfolge bevorzugt 3 bis unterhalb 8, 2 bis 7, 3 bis 7, 2,5 bis kleiner oder gleich 7, 2,5 bis 6 oder 4 bis 6. Das C₂/C₆-Verhältnis stellt insbesondere bei dem ersten Stärkederivat der erfindungsgemäßen Zusammensetzung einen weiteren Beitrag zur Lösung der der Erfindung zugrundeliegenden Aufgaben dar.

Die erfindungsgemäße Zusammensetzung weist mindestens zwei unterschiedliche Stärkederivate auf, die sich sowohl hinsichtlich ihres mittleren Molekulargewichts als auch hinsichtlich ihres molaren Substitutionsgrades unterscheiden. Üblicherweise weisen die Zusammensetzungen daher eine bimodale oder multimodale Molekulargewichtsverteilung auf. Bevorzugt sind Zusammensetzung bei denen das erste Stärkederivat und/oder das zweite Stärkederivat ein möglichst enge Molekulargewichtsverteilungskurve aufweist.

Im Rahmen der vorliegenden Erfindung ist das erste Stärkederivat ausgewählt aus der Gruppe bestehend aus Hydroxyalkylstärke, Carboxyalkylstärke, Esterstärke und beliebige Mischungen hiervon, wobei das Stärkederivat ein mittleres Molekulargewicht Mw1 und einen molaren Substitutionsgrad MS1 aufweist. Das mittlere Molekulargewicht Mw1 des ersten Stärkederivats ist größer als das mittlere Molekulargewicht Mw2 des zweiten Stärkederivats. Andererseits ist jedoch der molare Substitutionsgrad MS1 des ersten Stärkederivats kleiner als der molare Substitutionsgrad MS2 des zweiten Stärkederivats. Hierdurch wird eine optimale Plasmaverweildauer erzielt.

In dem Maß, wie das zweite Stärkederivat überwiegend renal ausgeschieden wird, können aus dem ersten Stärkederivat osmotisch wirksame Moleküle abgespalten werden.

In einer bevorzugten Ausführungsform weist das erste Stärkederivat ein mittleres Molekulargewicht von oberhalb 60000 Dalton, vorzugsweise 100000 bis 850000 Dalton, insbesondere 150000 bis 650000 Dalton auf.

Es hat sich als vorteilhaft für die Lösung der Aufgabe herausgestellt, dass der molare Substitutionsgrad MS1 des ersten Stärkederivats unterhalb von 0,35, vorzugsweise 0,05 bis 0,3 und insbesondere 0,1 bis 0,25 liegt.

In einer Ausgestaltung der vorliegenden Erfindung weist das erste Stärkederivat ein mittleres Molekulargewicht Mw1 von 80 und 500 kDa, und eine molare Substitution MS1 unter 0,5 auf. In einer vorteilhaften Ausgestaltung liegt Mw1 zwischen 100 und 300 kDa und MS1 unter 0.4. Ganz besonders vorteilhaft ist Mw1 zwischen 130 und 230 kDa und MS1 unter 0,3.

Im Rahmen der vorliegenden Erfindung ist das zweite Stärkederivat ausgewählt aus der Gruppe bestehend aus Hydroxyalkylstärke, Carboxyalkylstärke, Esterstärke und beliebige Mischungen hiervon, mit einem mittleren Molekulargewicht Mw2 und einem molaren Substitutionsgrad MS2.

Die erfindungsgemäße Zusammensetzung enthält das zweite Stärkederivat mit einem mittleren Molekulargewicht Mw2 von 30000 bis 80000 Dalton und insbesondere 40000 bis 70000 Dalton.

Bevorzugt weist das zweite Stärkederivat einen molaren Substitutionsgrad MS2 von 0,35 bis 0,8, weiter bevorzugt von 0,4 bis 0,7 und insbesondere von 0,5 bis 0,6 auf.
In einer bevorzugten Ausführungsform der Erfindung weist das zweite Stärkederivat ein mittleres Molekulargewicht Mw2 auf, welches unterhalb der Nierenschwelle, insbesondere beträgt Mw2 zwischen 5 kDa und 60 kDa und die molare Substitution MS2 liegt zwischen 0,5 und 0,9. In einer vorteilhaften Ausgestaltung hat das zweite Stärkederivat ein mittleres Molekulargewicht Mw2 zwischen 10 und 45 kDa und einen molaren Substitutionsgrad MS2 zwischen 0,6 und 0,8; besonders vorteilhaft ist Mw2 zwischen 20 und 40 kDa und MS2 zwischen 0,66 und 0,72.

Für bevorzugte erfindungsgemäße Zusammensetzungen ist das erste Stärkederivat und/oder das zweite Stärkederivat eine Hydroxyalkylstärke, insbesondere Hydroxyethylstärke.

Weiter bevorzugt gilt für die Zusammensetzung dass das erste Stärkederivat und/oder das zweite Stärkederivat eine Carboxyalkylstärke, vorzugsweise Carboxymethylstärke und/oder Carboxyethylstärke ist.

Geeignete Zusammensetzungen sind bevorzugt gekennzeichnet, dadurch dass das erste Stärkederivat und/oder das zweite Stärkederivat eine Esterstärke, insbesondere eine mit Mono- oder Dicarbonsäuren veresterte Stärke, im Speziellen Acetylstärke oder Propionylstärke ist.

Speziell bevorzugte Zusammensetzungen umfassen als zweites Stärkederivat eine Hydroxyethylstärke und als erstes Stärkederivat eine Hydroxyethylstärke oder Acetylstärke. Die erfindungsgemäßen Zusammensetzungen zeigen ein Gewichtsverhältnis von erstem Stärkederivat zu zweitem Stärkederivat von 1:9 bis 9:1, vorzugsweise 1:5 bis 8:1, besonders bevorzugt 1:3 bis 4:1 und insbesondere 1:1 bis 3:1.

Bevorzugt ist die Differenz Mw1-Mw2 mindestens 20000 Dalton, vorzugsweise mindestens 50000 Dalton und insbesondere mindestens 100000 Dalton, beispielsweise 150000 Dalton.
Weiter bevorzugt ist die Differenz MS2-MS1 mindestens 0,05, weiter bevorzugt mindestens 0,1, insbesondere mindestens 0,15 und im Speziellen mindestens 0,2, beispielsweise mindestens 0,3.

Bevorzugt sind Mischungen, bei denen der Gewichtsanteil des ersten Stärkederivats überwiegt. Die erfindungsgemäßen Zusammensetzungen zeigen bevorzugt eine bimodale oder auch multimodale Molekulargewichtsverteilung auf.

Die erfindungsgemäße pharmazeutische Zubereitung kann prinzipiell in jeder erdenklichen galenischen Darbietungsform bereitgestellt werden. In einer bevorzugten Ausführungsform der vorliegenden Erfindung können die erfindungsgemäßen pharmazeutischen Zubereitungen intravenös injiziert oder infundiert werden. Bevorzugt liegen die pharmazeutischen Zubereitungen daher als wässrige Lösung oder als kolloidale wässrige Lösung vor. Vorzugsweise enthalten die Zubereitungen die erfindungsgemäße Zusammensetzung in einer Konzentration von bis zu 20, weiter bevorzugt von 0,5 bis 15, besonders bevorzugt von 2 bis 12, insbesondere von 4 bis 10, beispielsweise 6 %.

Weiter bevorzugte Bereiche sind 2 bis 20%, weiter bevorzugt 4 bis 15% und insbesondere 6 bis 10%.

Die Mengenangaben erfolgen, wenn nicht anders angegeben in %, das im Rahmen der vorliegenden Erfindung gleichzusetzen ist mit g/100 ml Lösung.
Die erfindungsgemäßen pharmazeutischen Zubereitungen in Form von Kolloidlösungen stellen eine zur Einbringung in den menschlichen oder tierischen Körper geeignete Infusion zur Verfügung, die eine vorteilhafte steuerbare Erhöhung des osmotischen Drucks (Π) im fließenden Blut bewirkt und dabei einen minimalen Anteil an hochsubstituierten hochmolekularen Kolloiden im Körper zurückläßt.

In einer weiteren Ausführungsform enthalten die erfindungsgemäßen pharmazeutischen Zubereitungen zusätzlich Natriumchlorid, vorzugsweise 0,6 bis 2 %, besonders bevorzugt 0,9 %. Eine 0,9 prozentige Lösung von Natriumchlorid in Wasser wird auch als physiologische Kochsalzlösung bezeichnet. Sie weist den gleichen osmotischen Druck wie das Blutserum auf und ist daher als isotonische Lösung zur intravenösen Injektion oder Infusion geeignet. Zur Isotonisierung können auch alle anderen osmotisch wirksamen Substanzen verwendet werden, soweit sie physiologisch unbedenklich und gut verträglich sind wie z. B. Glucose, Glucoseersatzstoffe (Fructose, Sorbit, Xylit) oder Glycerin. Weiter können die Zubereitungen ein oder mehrere Komponenten ausgewählt aus der Gruppe bestehend aus Calciumchlorid, Calciumacetat, C2 bis C10- Mono-Alkansäuren oder -Polycarbonsäuren, (beispielsweise Propionsaure, Butansäure, Dicarbonsäuren, Oxalsäure, Malonsäure, Bernsteinsäure, Zitronensäure, Glutarsäure, Adipinsäure), Aminosäuren, (beispielsweise Glycin, Alanin, Prolin, Leucin, Isoleucin, Histidin), Acetessigsäure, β-Hydroxybutyrat, 3-Oxobutansäure, Acetoacetat, und Harnstoff aufweisen.

In einer weiteren bevorzugten Ausführungsform können die pharmazeutischen Zubereitungen zusätzlich weitere plasmaadaptierte Elektrolyte enthalten. Die Herstellung solcher isotoner Zubereitungen ist dem Fachmann bekannt. Ein Beispiel für eine isotone Lösung mit plasmaadaptierten Elektrolyten ist die sogenannte Tyrode-Lösung. Sie enthält 0,8 g NaCl, 0,02 g KCl, 0,02 g CaCl₂, 0,01 g MgCl₂, 0,005 g NaH₂PO₄, 0,1 g NaHCO₃ und 0,1 g Glucose in 100 ml destilliertem Wasser. Ein weiteres Beispiel ist die sogenannte Ringer-Lösung, die 0,8 % Natriumchlorid, 0,02 % Kaliumchlorid, 0,02 % Calciumchlorid und 0,1 % Natriumhydrogencarbonat aufweist. Hierbei können selbstverständlich auch die Anionen der Elektrolyte durch metabolisierbare Anionen ausgetauscht werden, so kann beispielsweise in der Ringer-Lösung das Natriumhydrogencarbonat durch 0,3 oder 0,6 % Natriumlactat ersetzt werden. Eine entsprechende Elektrolytzusammensetzung bzw. -lösung ist dem Fachmann als "Ringer-Lactat" bekannt. Weitere metabolisierbare Anionen, die allein oder in Kombination Verwendung finden können, sind Acetat (z.B. "Ringer-Acetat") oder Malat.

In einer weiteren Ausführungsform der Erfindung können die pharmazeutischen Zubereitungen auch als hypertone Lösungen vorliegen. Hypertone Lösungen sind solche mit einem höheren osmotischen Druck als das menschliche Blut. Die Applikation hypertoner pharmazeutischer Zubereitungen kann bei bestimmten Krankheitsbildern vorteilhaft sein. Der erforderliche hohe osmotische Druck hypertoner Lösungen wird durch Zugabe entsprechender Mengen osmotisch wirksamer Substanzen eingestellt, z.B. durch Natriumchlorid, das zu diesem Zweck in Konzentrationen bis 7,5 % und mehr verwendet werden kann.

Gerade im Zusammenspiel mit Elektrolyten zeigen die erfindungsgemäßen pharmazeutischen Zubereitungen in Form von wässerigen Formulierungen, beispielsweise Infusionslösungen erhebliche Vorteile gegenüber den HES basierten Zubereitungen des Standes der Technik. Es hat sich nämlich überraschend gezeigt, dass sich die erfindungsgemäßen Zubereitungen wesentlich effektiver in ihrem Viskositätsverhalten durch Zugabe von Elektrolyten steuern lassen. Dies kann beispielsweise auch zu Diagnosezwecken genutzt werden, nachdem die Zubereitung infundiert wurde.

Zur Vermeidung und Reduzierung der Gefahr von Infektionen werden die erfindungsgemäßen pharmazeutischen Zubereitungen vorzugsweise sterilfiltriert oder hitzesterilisiert. Zwecks Sterilfiltration erfindungsgemäßer wässriger oder kolloidaler wässriger pharmazeutischer Zubereitungen eignen sich insbesondere feinporige Filterkartuschen, wie sie beispielsweise unter dem Markennamen SARTPORE von der Firma Sartorius zur Verfügung gestellt werden. Geeignet sind beispielsweise solche Filterkartuschen mit einem Porendurchmesser von 0,2 µm. Die erfindungsgemäßen pharmazeutischen Zubereitungen können darüber hinaus hitzesterilisiert werden, ohne dass es zu einer Beeinträchtigung der Stärkederivate der Zusammensetzung kommt. Vorzugsweise wird die Hitzesterilisation bei einer Temperatur oberhalb von 100 °C, besonders bevorzugt zwischen 105 und 150 °C, insbesondere zwischen 110 und 130 °C, beispielsweise 121 °C über einen Zeitraum von bis zu 30 Minuten, vorzugsweise bis zu 25 Minuten, insbesondere zwischen 18 und 22 Minuten durchgeführt.

In einer bevorzugten Ausführungsform ist die pharmazeutische Zubereitung ein Volumenersatzmittel. Volumenersatzmittel finden ihren Einsatz beim Ersatz intravasaler Flüssigkeit in tierischen und menschlichen Organismen. Volumenersatzmittel finden insbesondere bei der Prophylaxe und Therapie der Hypovolämie ihren Einsatz. Es ist dabei unbedeutend, ob die Hypovolämie aus dem unmittelbaren Verlust von Blut bzw. Körperflüssigkeiten resultiert, wie beispielsweise bei akuten Blutungen, Traumen, Operationen, Verbrennungen, usw. oder aus Verteilungsstörungen zwischen Makro- und Mikrozirkulation, wie beispielsweise bei der Sepsis oder aus einer Vasodilatation, wie beispielsweise bei der Einleitung der Anästhesie. Die Volumenersatzmittel werden dabei weiter unterteilt in die sogenannten Plasmaersatzmittel und die sogenannten Plasmaexpander. Bei den Plasmaersatzmitteln entspricht das intravasal applizierte Volumen des Mittels auch dem Volumen, das den Gefäßen zugeführt wird. Bei den Plasmaexpandern hingegen ist das intravasal applizierte Flüssigkeitsvolumen des Expanders geringer als das den Gefäßen real zugeführte Volumen. Dieses Phänomen liegt darin begründet, dass durch den Einsatz von Plasmaexpandern das onkotische Gleichgewicht zwischen Intra- und Extravasalraum gestört wird und zusätzliches Flüssigkeitsvolumen in das zu behandelnde Gefäßsystem aus dem extravasalen Raum einströmt.

Plasmaexpander unterscheiden sich von den Plasmaersatzmitteln im Wesentlichen dadurch, dass die Konzentration der enthaltenen erfindungsgemäßen Zusammensetzung erhöht ist und/oder die Konzentration der jeweiligen Elektrolyte ein onkotisches und/oder osmotisches Ungleichgewicht hervorrufen.

Die erfindungsgemäße pharmazeutische Zubereitung kann ferner einen pharmazeutischen Wirkstoff oder Wirkstoffkombinationen enthalten und so als Medium zur Verabreichung der darin gelösten Wirkstoffe, insbesondere durch Injektion und Infusion dienen.

Ein weiterer Gegenstand der vorliegenden Erfindung ist die Verwendung einer erfindungsgemäßen pharmazeutischen Zubereitung zur Herstellung eines Volumenersatzmittels oder eines Plasmaersatzmittels oder eines Plasmaexpanders.

Die erfindungsgemäßen pharmazeutischen Zubereitungen können besonders bevorzugt als Volumenersatzmittel oder Plasmaersatzmittel oder Plasmaexpander verwendet werden. Bevorzugt dienen die pharmazeutischen Zubereitungen der Aufrechterhaltung der Normovolämie. Die Auftrechterhaltung der Normovolämie ist von besonderer Bedeutung für die hämodynamische Stabilität, die einen entscheidenden Einfluss auf den menschlichen oder tierischen Organismus, beispielsweise bezüglich des Blutdrucks, der Diureserate oder der Herzfrequenz hat. Um die Aufrechterhaltung der Normovolämie möglichst schnell nach einem intravasalen Flüssigkeitsverlust zu kompensieren, haben sich die erfindungsgemäßen pharmazeutischen Zubereitungen als besonders vorteilhaft erwiesen, da sie im Vergleich zu den im Stand der Technik bekannten Plasmaersatzmitteln, speziell niedermolekularen HES-Lösungen, wie z.B. HES 130/0,4, eine verlängerte Plasmahalbwertszeit aufweisen, insbesondere in der entscheidenden Phase unmittelbar nach der Infusion. Die Plasmaviskosität kann mit Hilfe der erfindungsgemäßen pharmazeutischen Zubereitung sehr gut gesteuert und reguliert werden und überraschenderweise nicht ansteigt, sorgt dabei ebenfalls für eine Verbesserung der Mikrozirkulation sowie zu einer verbesserten nutritiven Sauerstoffversorgung der Gewebe.

Ein weiterer Gegenstand ist die Verwendung der erfindungsgemäßen pharmazeutischen Zubereitung zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten.

Die erfindungsgemäßen pharmazeutischen Zubereitungen oder die erfindungsgemäße Zusammensetzung werden vorzugsweise zur Herstellung von Medikamenten, insbesondere für Medikamente zur Aufrechterhaltung der Normovolämie und/oder zur Verbesserung der Makro- und Mikrozirkulation und/oder zur Verbesserung der nutritiven Sauerstoffversorgung und/oder zur Stabilisierung der Hämodynamik und/oder zur Verbesserung der Volumenwirksamkeit und/oder zur Verringerung der Plasmaviskosität und/oder zur Erhöhung der Anämietoleranz und/oder zur Hämodilution, insbesondere zur therapeutischen Hämodilution bei Durchblutungsstörungen und arteriellen, speziell peripheren arteriellen, Verschlusskrankheiten verwendet.

Darüber hinaus werden die erfindungsgemäßen pharmazeutischen Zubereitungen oder die erfindungsgemäßen Zubereitungen vorteilhafterweise in Verfahren zur Behandlung der Aufrechterhaltung der Normovolämie und/oder Verbesserung der Makro- und Mikrozirkulation und/oder Verbesserung der nutritiven Sauerstoffversorgung und/oder Stabilisierung der Hämodynamik und/oder Verbesserung der Volumenwirksamkeit und/oder Verringerung der Plasmaviskosität und/oder Erhöhung der Anämietoleranz und/oder Hämodilution, insbesondere therapeutischen Hämodilution bei Durchblutungsstörungen und arteriellen, speziell peripheren arteriellen, Verschlusskrankheiten eingesetzt.

Die Einflussnahme der erfindungsgemäßen Zusammensetzungen bzw. der pharmazeutischen Zubereitungen auf den Magnesiumhaushalt machen diese auch für Arzneimittel und Diagnostika geeignet.

Ein weiterer Gegenstand der Erfindung ist die erfindungsgemäße pharmazeutische Zubereitung zur Verwendung in der Prophylaxe oder Therapie von Erkrankungen, ausgewählt aus der Gruppe bestehend aus Krankheiten mit erniedrigten kolloidosmotischen Druck, Blutungsanämie, Schock, SIRS/Sepsis, Thrombolyse, Apoplexie und Eklampsie.

Ein weiterer Gegenstand ist die Verwendung der erfindungsgemäßen Zubereitung zur Behandlung von Dehydratation, Flüssigkeits- und Elektrolytmangel, Eiweißmangel. Des Weiteren kann die Zusammensetzung verwendet werden als Ersatz von Blutbestandteilen, Trägerlösung für Arzneistoffe, Mittel zur Verbesserung der thermodynamischen Güte von Blutplasma /oder Blutserum, Mittel zur Veränderung des Brechungsinkrements des Blutes, Ernährungslösung, Rheologica, Mittel zur Verbesserung der Organdurchblutung, Thromboseprophylaxe, Adjuvans zur Thrombolyse, Venotonikum, Mittel zur Verbesserung der Wirksamkeit von Antiarrhythmika und Mittel zur Applikation von Magnesium.

Ein weiterer Gegenstand ist ein Verfahren zur Herstellung einer erfindungsgemäßen Zusammensetzung wie in Anspruch 14 definiert.

Es hat sich überraschend gezeigt, dass sich Stärkederivate mit niedrigem Molekulargewicht aus hochmolekularen Stärkederivaten durch Ultraschallabbau herstellen lassen. Daher ist es bevorzugt, dass das zweite Stärkederivat durch Ultraschallabbau erhältlich ist.

In einer bevorzugten Ausgestaltung können die erfindungsgemäße Zusammensetzung erhalten werden durch verzögerte Zugabe von Stärkemolekülen mit aufsteigendem Molekulargewicht während des Prozess der chemischen Einbringung des Substituenten (Hydroxyethylierung, Carboxymethylierung, Acetylierung). Hierdurch werden Kolloide mit größerem Molekulargewicht mit entsprechend niedrigeren molaren Substitutionen versehen.

### Beispiel 1:

Mit dem von De Belder und Granath (Carbohydrate Research, 30 (1973) 375-378) beschriebenen Verfahren wird fluoreszenzmarkierte Hydroxyethylstärke (FITC-HES) mit einem mittleren Molekulargewicht Mw von 40 kDa und einem molaren Substitutionsgrad MS von 0,5 hergestellt.
A.: 4 ml einer wässrigen 6%-igen FITC-HES 40.000/0,5 werden mit 6ml einer wässrigen NaCl-Lösung (0,9%-ig) gemischt. Die Lösung wird 3 WISTAR Ratten über einen Zentralvenenkatheter infundiert.
B.: 4 ml der 6%ige FITC-HES 40.000/0,5 und 6 ml einer wässrigen (10%ige) Acetylstärke-Lösung (200.000 Dalton; MS 0,3) werden ebenfalls 3 WISTAR Ratten via Zentralvenenkatheter infundiert.

In beiden Gruppen wird der Urin der ersten Stunde aufgefangen und mit einem Fluoreszenzdetektor untersucht. Es zeigt sich, dass 60 Minuten nach Infusion die im Urin gemessene Fluoreszenz (585nm) bei den Tieren, die die 4 ml 6%ige FITC-HES 40.000/0,5 + 6ml Acetylstärke 200.000 MS 0,3 erhalten hatten um 15- 20% unter der Fluoreszenzausscheidung der Tiere lag, die die 4 ml 6%-ige FITC-HES 40.000/0,5 + 6ml NaCl 0,9% erhalten hatten. Nach beiden Infusionen wurden vergleichbare Fluoreszenzaufnahmen in zirkulierende Blutzellen mittels Flow-Cytometrie gemessen.

### Beispiel 2:

Eine Hydroxyethylstärkemischung (1), enthaltend 33,3 Gewichtsprozent einer Hydroxyethylstärke (Mw: 40 kDa; MS = 0,55) wird mit 66,6 Gewichtsprozent einer Hydroxyethylstärke (Mw: 130 kDa; MS = 0,4) gemischt.
**A:** Es werden unterschiedlich konzentrierte Dispersionen dieser Hydroxyethylstärkemischung (1) in einer Kochsalzlösung (0,9%-ige NaCl-Lösung, enthaltend 154 mmol/l Na⁺ und 154 mmol/l Cl⁻) mit den folgenden Konzentrationen hergestellt: 2% (2 g/100 ml), 4% (4 g/100 ml), 6% (6 g/100 ml), 8% (8 g/100 ml) und 10% (10 g/100 ml).
**B:** Es werden unterschiedlich konzentrierte Dispersionen dieser Hydroxyethylstärkemischung (1) in einer Kochsalzlösung, die zusätzlich Magnesiumacetat enthält (6 mmol/l Magnesiumacetat-tetrahydrat; NaCI-Lösung, enthaltend 136 mmol/l Na⁺ und 136 mmol/l Cl⁻), mit den folgenden Konzentrationen hergestellt: 2% (2 g/100 ml), 4% (4 g/100 ml), 6% (6 g/100 ml), 8% (8 g/100 ml) und 10% (10 g/100 ml).

Die 6%ige Lösung weist einen mit einer 10 kDa Membran gemessenen osmotischen Druck von 53-58 mmHg auf.

Die Viskositäten der unter A und B hergestellten kolloiden Dispersionen werden bei unterschiedlichen Schergeschwindigkeiten mit einem HAAKE MARS Rheometer bei 310°K und einer Spaltbreite von 0,052 mm gemessen.

### 2.1 Untersuchung der Zubereitungen mit einer Konzentration von 2 % bei unterschiedlichen Schergeschwindigkeiten.

Bei kleinerer Schergeschwindigkeit 56,4/s wurden bei der 2%-igen Lösungen A eine Viskosität von 1,11 mPas und bei der entsprechend 2%-igen Lösung B eine Viskosität von 1,44 mPas gemessen.

Bei höheren Schergeschwindigkeiten 117/s zeigt die Lösung A eine Viskosität von 1,07 mPas und die Lösung B eine Viskosität von 1,15mPas auf.

Bei einer Schergeschwindigkeit von 500/s zeigen beide Lösungen gleiche Viskositäten auf, nämlich für A: 1,01mPas und für B:1,01mPas.

### 2.2 Untersuchung der Zubereitungen mit einer Konzentration von 4 % bei unterschiedlichen Schergeschwindigkeiten.

Auch bei der 4%-igen Dispersionen traten bei niedrigeren Schergeschwindigkeiten größere Unterschiede zwischen Dispersion A und Dispersion B auf: Bei Dispersion A wurden bei einer Schergeschwindikgeit von 56,4/s eine Viskosität von 1,47 mPas und bei B wurde eine Viskosität von 1,75 mPas gemessen.

Bei 500/s zeigte A eine Viskosität von 1,34 mPas und B eine Viskosität von 1,36 mPas auf.

### 2.3 Untersuchung der Zubereitungen mit einer Konzentration von 8 % bei unterschiedlichen Schergeschwindigkeiten.

Bei einer Konzentration von 8 % wurden die folgenden Viskositätswerte gemessen:
Schergeschwindigkeit: 56,4/s. Die Dispersion A zeigt eine Viskosität von 2,35 mPas und Dispersion B von 2,60 mPas.
Schergeschwindigkeit: 117/s. Die Dispersion A zeigt eine Viskosität von 2,38 mPas und Dispersion B von 2,42 mPas.
Schergeschwindigkeit: 500/s. Die Dispersion A zeigt eine Viskosität von 2,41 mPas und Dispersion B von 2,43 mPas.

### 2.4 Untersuchung der Zubereitungen mit einer Konzentration von 10 % bei unterschiedlichen Schergeschwindigkeiten.

Bei einer Konzentration von 10 % wurden die folgenden Viskositätswerte gemessen:
Schergeschwindigkeit: 56,4/s. Die Dispersion A zeigt eine Viskosität von 2,78 mPas und Dispersion B von 2,97 mPas.
Schergeschwindigkeit: 117/s. Die Dispersion A zeigt eine Viskosität von 2,79 mPas und Dispersion B von 2,94 mPas.
Schergeschwindigkeit: 500/s. Die Dispersion A zeigt eine Viskosität von 2,81 mPas und Dispersion B von 2,97 mPas.

Bei 10%igen Konzentrationen blieben die Viskositäten der Dispersion B kontinuierlich und deutlich über den Werten der A Dispersionen.

Die Ergebnisse belegen die überraschend hohe Einflussnahme der Elektrolyte auf das Viskosiätsverhalten. Insbesondere bei Einsatz von Magnesium-haltigen Elektrolyten kann die Viskosität der kolloidalen Infusionslösungen gut gesteuert werden.

## Patentansprüche

1. Wässrige pharmazeutische Zubereitung umfassend
i) eine Zusammensetzung umfassend
a) ein erstes Stärkederivat ausgewählt aus der Gruppe bestehend aus Hydroxyalkylstärke, Carboxyalkylstärke, Esterstärke und beliebige Mischungen hiervon, wobei das Stärkederivat ein mittleres Molekulargewicht Mw1 und einen molaren Substitutionsgrad MS1 aufweist und
b) ein zweites Stärkederivat ausgewählt aus der Gruppe bestehend aus Hydroxyalkylstärke, Carboxyalkylstärke, Esterstärke und beliebige Mischungen hiervon, mit einem mittleren Molekulargewicht Mw2 und einem molaren Substitutionsgrad MS2, wobei Mw1 größer als Mw2 (Mw1 > Mw2) und MS2 größer als MS1 (MS2 > MS1)ist, wobei das Gewichtsverhältnis von erstem Stärkederivat zu zweitem Stärkederivat 1:9 bis 9:1 ist und wobei das zweite Stärkederivat ein mittleres Molekulargewicht Mw2 von 30000 bis 80000 Dalton, bevorzugt 40000 bis 70000 Dalton aufweist; und
ii) ein oder mehrere Elektrolyte.

2. Pharmazeutische Zubereitung gemäß Anspruch 1, **dadurch gekennzeichnet, dass** das zweite Stärkederivat einen molaren Substitutionsgrad MS2 von 0,35 bis 0,8, vorzugsweise von 0,4 bis 0,7 und insbesondere von 0,5 bis 0,6 aufweist.

3. Pharmazeutische Zubereitung gemäß Anspruch 1, **dadurch gekennzeichnet, dass** das erste Stärkederivat ein mittleres Molekulargewicht von oberhalb 60000 Dalton, vorzugsweise 100000 bis 850000 Dalton, insbesondere 150000 bis 650000 Dalton aufweist, und/oder dass der molare Substitutionsgrad MS1 des ersten Stärkederivats unterhalb von 0,35, vorzugsweise 0,05 bis 0,3 und insbesondere 0,1 bis 0,25 beträgt.

4. Pharmazeutische Zubereitung gemäß mindestens einem der vorangehenden Ansprüche, **dadurch gekennzeichnet, dass** das erste Stärkederivat und/oder das zweite Stärkederivat eine Hydroxyalkylstärke, insbesondere Hydroxyethylstärke ist, oder dass das erste Stärkederivat und/oder das zweite Stärkederivat eine Carboxyalkylstärke, vorzugsweise Carboxymethylstärke und/oder Carboxyethylstärke ist, oder dass das erste Stärkederivat und/oder das zweite Stärkederivat eine Esterstärke, insbesondere eine mit Mono- oder Dicarbonsäuren veresterte Stärke, im Speziellen Acetylstärke oder Propionylstärke ist.

5. Pharmazeutische Zubereitung gemäß mindestens einem der vorangehenden Ansprüche, **dadurch gekennzeichnet, dass** die derivatisierte Stärke auf zumindest 50 Gew.-%, vorzugsweise mindestens 65 Gew.-% und insbesondere mindestens 75 Gew.-% auf Amylose basiert.

6. Pharmazeutische Zubereitung gemäß mindestens einem der vorangehenden Ansprüche, **dadurch gekennzeichnet, dass** das Gewichtsverhältnis von erstem Stärkederivat zu zweitem Stärkederivat 1:5 bis 8:1, besonders bevorzugt 1:3 bis 4:1 und insbesondere 1:1 bis 3:1 ist.

7. Pharmazeutische Zubereitung gemäß Anspruch 1, **dadurch gekennzeichnet, dass** die Zubereitung wässrig ist und die Zusammensetzung in einer Menge von 2 bis 20%, vorzugsweise 4 bis 15 Gew.-% und insbesondere 6 bis 10 Gew.-%, vorliegt.

8. Pharmazeutische Zubereitung gemäß mindestens einem der Ansprüche 1 bis 7, **dadurch gekennzeichnet, dass** sie zusätzlich ein oder mehrere Elektrolyte, ausgewählt aus der Gruppe bestehend aus Natriumchlorid, Kaliumchlorid, Magnesiumacetat und Magnesiumchlorid enthält oder insbesondere Magnesium-haltige Elektrolyte, und/oder dass die Zubereitung ein oder mehrere Komponenten ausgewählt aus der Gruppe bestehend aus Calciumchlorid, Calciumacetat, C2 bis C10- Mono-Alkansäuren oder -Polycarbonsäuren, (beispielsweise Propionsaure, Butansäure, Dicarbonsäuren, Oxalsäure, Malonsäure, Bernsteinsäure, Zitronensäure, Glutarsäure, Adipinsäure), Aminosäuren, (beispielsweise Glycin, Alanin, Prolin, Leucin, Isoleucin, Histidin), Acetessigsäure, β-Hydroxybutyrat, 3-Oxobutansäure, Acetoacetat, und Harnstoff enthält.

9. Pharmazeutische Zubereitung gemäß mindestens einem der Ansprüche 1 bis 8, **dadurch gekennzeichnet, dass** die Zubereitung isoton ist.

10. Pharmazeutische Zubereitung gemäß mindestens einem der Ansprüche 1 bis 9, **dadurch gekennzeichnet, dass** sie ein Volumenersatzmittel ist.

11. Pharmazeutische Zubereitung gemäß mindestens einem der Ansprüche 1 bis 10, **dadurch gekennzeichnet, dass** sie einen pharmazeutischen Wirkstoff oder eine Wirkstoffkombination enthält.

12. Pharmazeutische Zubereitung gemäß mindestens einem der Ansprüche 1 bis 11 zur Verwendung als Plasmaersatzmittel oder Plasmaexpander oder zur Verwendung in der Prophylaxe oder Therapie von Erkrankungen, ausgewählt aus der Gruppe bestehend aus Blutungsanämie, Schock, SIRS/Sepsis, Thrombolyse, Apoplexie, Dehydratation, Flüssigkeits- und Elektrolytmangel, Eiweißmangel und Eklampsie.

13. Pharmazeutische Zubereitung gemäß mindestens einem der Ansprüche 1 bis 12 zur Verwendung als Ersatz von Blutbestandteilen, Trägerlösung für Arzneistoffe, Mittel zur Verbesserung der thermodynamischen Güte von Blutplasma /oder Blutserum, Ernährungslösung, Rheologica, Mittel zur Verbesserung der Organdurchblutung, Thromboseprophylaxe, Adjuvans zur Thrombolyse, Venotonikum, Mittel zur Verbesserung der Wirksamkeit von Antiarrhythmika und Mittel zur Applikation von Magnesium.

14. Verfahren zur Herstellung einer Zusammensetzung gemäß mindestens einem der Ansprüche 1-13, umfassend die folgenden Schritte:
a) Bereitstellen eines ersten Stärkederivats, ausgewählt aus der Gruppe bestehend aus Hydroxyalkylstärke, Carboxyalkylstärke, Esterstärke und beliebige Mischungen hiervon, wobei das Stärkederivat ein mittleres Molekulargewicht Mw1 und einen molaren Substitutionsgrad MS1 aufweist,
b) Bereitstellen eines zweiten Stärkederivats, ausgewählt aus der Gruppe bestehend aus Hydroxyalkylstärke, Carboxyalkylstärke, Esterstärke und beliebige Mischungen hiervon mit einem mittleren Molekulargewicht Mw2 und einem molaren Substitutionsgrad MS2, wobei Mw1 größer als Mw2 (Mw1 > Mw2) und MS2 größer als MS1 (MS2 > MS1) ist, wobei das Gewichtsverhältnis von erstem Stärkederivat zu zweitem Stärkederivat 1:9 bis 9:1 und wobei das zweite Stärkederivat ein mittleres Molekulargewicht Mw2 von 30000 bis 80000 Dalton aufweist,
c) Mischen des ersten Stärkederivats mit dem zweiten Stärkederivat, und
d) Zugabe eines oder mehrerer Elektrolyte.

## Claims

1. An aqueous pharmaceutical formulation, comprising
i) a composition comprising
a) a first starch derivative selected from the group consisting of hydroxyalkyl starch, carboxyalkyl starch, ester starch and any mixtures thereof, wherein said starch derivative has an average molecular weight Mw1 and a molar degree of substitution MS1; and
b) a second starch derivative selected from the group consisting of hydroxyalkyl starch, carboxyalkyl starch, ester starch and any mixtures thereof with an average molecular weight Mw2 and a molar degree of substitution MS2, wherein Mw1 is greater than Mw2 (Mw1 > Mw2), and MS2 is greater than MS1 (MS2 > MS1), wherein the weight ratio of the first starch derivative to the second starch derivative is 1:9 to 9:1, and wherein the second starch derivative has an average molecular weight Mw2 of 30,000 to 80,000 daltons, preferably from 40,000 to 70,000 daltons; and
ii) one or more electrolytes.

2. The pharmaceutical formulation according to claim 1, **characterized in that** said second starch derivative has a molar degree of substitution MS2 of 0.35 to 0.8, preferably from 0.4 to 0.7, and especially from 0.5 to 0.6.

3. The pharmaceutical formulation according to claim 1, **characterized in that** said first starch derivative has an average molecular weight above 60,000 daltons, preferably from 100,000 to 850,000 daltons, especially from 150,000 to 650,000 daltons, and/or that the molar degree of substitution MS1 of the first starch derivative is below 0.35, preferably from 0.05 to 0.3, and especially from 0.1 to 0.25.

4. The pharmaceutical formulation according to at least one of the preceding claims, **characterized in that** said first starch derivative and/or said second starch derivative is a hydroxyalkyl starch, especially a hydroxyethyl starch, or that said first starch derivative and/or said second starch derivative is a carboxyalkyl starch, preferably carboxymethyl starch and/or carboxyethyl starch, or that said first starch derivative and/or said second starch derivative is an ester starch, especially a starch esterified with mono- or dicarboxylic acids, more particularly acetyl starch or propionyl starch.

5. The pharmaceutical formulation according to at least one of the preceding claims, **characterized in that** the derivatized starch is based on at least 50% by weight, preferably at least 65% by weight, and especially at least 75% by weight, of amylose.

6. The pharmaceutical formulation according to at least one of the preceding claims, **characterized in that** the weight ratio of the first starch derivative to the second starch derivative is 1:5 to 8:1, more preferably from 1:3 to 4:1, and especially from 1.1 to 3:1.

7. The pharmaceutical formulation according to claim 1, **characterized in that** said formulation is aqueous in nature, and that the composition is present in an amount of from 2 to 20%, preferably from 4 to 15% by weight, and especially from 6 to 10% by weight.

8. The pharmaceutical formulation according to at least one of claims 1 to 7, **characterized in that** it additionally contains one or more electrolytes selected from the group consisting of sodium chloride, potassium chloride, magnesium acetate and magnesium chloride, or especially magnesium-containing electrolytes, and/or that the formulation contains one or more components selected from the group consisting of calcium chloride, calcium acetate, C₂ to C₁₀ monoalkanoic acids or polycarboxylic acids (for example, propionic acid, butanoic acid, dicarboxylic acids, oxalic acid, malonic acid, succinic acid, citric acid, glutaric acid, adipic acid), amino acids (for example, glycine, alanine, proline, leucine, isoleucine, histidine), acetoacetic acid, β-hydroxybutyrate, 3-oxobutanoic acid, acetoacetate, and urea.

9. The pharmaceutical formulation according to at least one of claims 1 to 8, **characterized in that** said formulation is isotonic.

10. The pharmaceutical formulation according to at least one of claims 1 to 9, **characterized by** being a volume replacement.

11. The pharmaceutical formulation according to at least one of claims 1 to 10, **characterized by** containing a pharmaceutically active substance or a combination of active substances.

12. The pharmaceutical formulation according to at least one of claims 1 to 11 for use as a plasma replacement or plasma expander, or for use in the prophylaxis and therapy of diseases selected from the group consisting of acute blood loss anemia, shock, SIRS/sepsis, thrombolysis, apoplexy, dehydration, liquid and electrolyte deficiencies, protein deficiency, and eclampsia.

13. The pharmaceutical formulation according to at least one of claims 1 to 12 for use as a replacement of blood components, carrier solution for medicinal substances, agent for improving the thermodynamic quality of blood plasma or blood serum, nutritional solution, rheological modifiers, agent for improving the blood supply to organs, thrombosis prophylaxis, adjuvant for thrombolysis, venotonic, agent for improving the efficiency of antiarrhythmics, and agent for administering magnesium.

14. A process for preparing a composition according to at least one of claims 1 to 13, comprising the following steps:
a) providing a first starch derivative selected from the group consisting of hydroxyalkyl starch, carboxyalkyl starch, ester starch and any mixtures thereof, wherein said starch derivative has an average molecular weight Mw1 and a molar degree of substitution MS1;
b) providing a second starch derivative selected from the group consisting of hydroxyalkyl starch, carboxyalkyl starch, ester starch and any mixtures thereof with an average molecular weight Mw2 and a molar degree of substitution MS2, wherein Mw1 is greater than Mw2 (Mw1 > Mw2), and MS2 is greater than MS1 (MS2 > MS1), wherein the weight ratio of the first starch derivative to the second starch derivative is 1:9 to 9:1, and wherein the second starch derivative has an average molecular weight Mw2 of 30,000 to 80,000 daltons;
c) mixing said first starch derivative with said second starch derivative; and
d) adding one or more electrolytes.

## Revendications

1. Formulation pharmaceutique aqueuse, comprenant
i) une composition comprenant
a) un premier dérivé d'amidon choisi dans le groupe consistant en amidon hydroxyalkylé, amidon carboxyalkylé, ester d'amidon et des mélanges quelconques de ceux-ci, dans laquelle ledit dérivé d'amidon présente un poids moléculaire moyen Mw1 et un degré de substitution molaire MS1, et
b) un second dérivé d'amidon choisi dans le groupe consistant en amidon hydroxyalkylé, amidon carboxyalkylé, ester d'amidon et des mélanges quelconques de ceux-ci, ayant un poids moléculaire moyen Mw2 et un degré de substitution molaire MS2, où Mw1 est supérieur à Mw2 (Mw1 > Mw2), et MS2 est supérieur à MS1 (MS2 > MS1), dans laquelle le rapport pondéral du premier dérivé d'amidon au second dérivé d'amidon est de 1:9 à 9:1, et dans laquelle ledit second dérivé d'amidon présente un poids moléculaire moyen Mw2 de 30000 à 80000 daltons, de préférence de 40000 à 70000 daltons, et
ii) un ou plusieurs électrolytes.

2. Formulation pharmaceutique selon la revendication 1, **caractérisée en ce que** ledit second dérivé d'amidon présente un degré de substitution molaire MS2 de 0,35 à 0,8, de préférence de 0,4 à 0,7, et spécifiquement de 0,5 à 0,6.

3. Formulation pharmaceutique selon la revendication 1, **caractérisée en ce que** ledit premier dérivé d'amidon présente un poids moléculaire moyen de plus de 60000 daltons, de préférence de 100000 à 850000 daltons, spécifiquement de 150000 à 650000 daltons, et/ou que le degré de substitution molaire MS1 dudit premier dérivé d'amidon est moins de 0,35, de préférence de 0,05 à 0,3, et spécifiquement de 0,1 à 0,25.

4. Formulation pharmaceutique selon l'une au moins des revendications précédentes, **caractérisée en ce que** ledit premier dérivé d'amidon et/ou ledit second dérivé d'amidon est un amidon hydroxyalkylé, spécifiquement un amidon hydroxyéthylé, ou que ledit premier dérivé d'amidon et/ou ledit second dérivé d'amidon est un amidon carboxyalkylé, spécifiquement un amidon carboxyméthylé et/ou un amidon carboxyéthylé, ou que ledit premier dérivé d'amidon et/ou ledit second dérivé d'amidon est un ester d'amidon, spécifiquement un amidon estérifié avec des acides mono- ou dicarboxyliques, plus spécifiquement un amidon acétylé ou un amidon propionylé.

5. Formulation pharmaceutique selon l'une au moins des revendications précédentes, **caractérisée en ce que** l'amidon dérivatisé est basé sur au moins 50% en poids, de préférence au moins 65% en poids, et notamment au moins 75% en poids d'amylose.

6. Formulation pharmaceutique selon l'une au moins des revendications précédentes, **caractérisée en ce que** le rapport pondéral dudit premier dérivé d'amidon audit second dérivé d'amidon est de 1:5 à 8:1, de préférence de 1:3 à 4:1, et spécifiquement de 1:1 à 3:1.

7. Formulation pharmaceutique selon la revendication 1, **caractérisée en ce que** ladite formulation est aqueuse, et que ladite composition est présente en une quantité de 2 à 20%, de préférence de 4 à 15% en poids, et spécifiquement de 6 à 10% en poids.

8. Formulation pharmaceutique selon l'une au moins des revendications 1 à 7, **caractérisée en ce qu'**elle contient en outre un ou plusieurs électrolytes choisis dans le groupe consistant en chlorure de sodium, chlorure de potassium, acétate de magnésium et chlorure de magnésium, ou spécifiquement des électrolytes contenant du magnésium, et/ou que ladite formulation contient un ou plusieurs composants choisis dans le groupe consistant en chlorure de calcium, acétate de calcium, des acides monoalcanoïques ou poly-carboxyliques en C2 à C10 (par exemple, l'acide propionique, l'acide buta-noïque, des acides dicarboxyliques, l'acide oxalique, l'acide malonique, l'acide succinique, l'acide citrique, l'acide glutarique, l'acide adipique), des acides aminés (par exemple, la glycine, l'alanine, la proline, la leucine, l'isoleucine, l'histidine), l'acide acétoacétique, le β-hydroxybutyrate, l'acide 3-oxobutanoïque, l'acétoacétate, et l'urée.

9. Formulation pharmaceutique selon l'une au moins des revendications 1 à 8, **caractérisée en ce que** ladite formulation est isotonique.

10. Formulation pharmaceutique selon l'une au moins des revendications 1 à 9, **caractérisée en ce qu'**elle est un agent de remplacement du volume sanguin.

11. Formulation pharmaceutique selon l'une au moins des revendications 1 à 10, **caractérisée en ce qu'**elle contient un agent pharmaceutiquement actif ou une combinaison d'agents pharmaceutiquement actifs.

12. Formulation pharmaceutique selon l'une au moins des revendications 1 à 11 destinée à être utilisée en tant qu'agent de remplacement du plasma ou expanseur du volume plasmatique, ou destinée à être utilisée dans la prophylaxie ou thérapie de maladies choisies dans le groupe consistant en anémie aiguë post-hémorragique, choc, SIRS/septicémie, thrombolyse, apoplexie, déshydratation, manque de liquide et d'électrolytes, manque de protéines, et éclampsie.

13. Formulation pharmaceutique selon l'une au moins des revendications 1 à 12 destinée à être utilisée en tant que remplacement de composants sanguins, solution porteuse pour substances médicamenteuses, agent pour améliorer la qualité thermodynamique de plasma sanguin ou de sérum sanguin, solution nutritionnelle, agent rhéologique, agent pour améliorer l'irrigation sanguine des organes, la prophylaxie de la thrombose, adjuvant pour la thrombolyse, agent veinotonique, agent pour améliorer l'efficacité d'agents antia-rythmiques, et agent pour l'administration de magnésium.

14. Procédé pour la préparation d'une composition selon l'une au moins des revendications 1 à 13, comprenant les étapes suivantes consistant à :
a) procurer un premier dérivé d'amidon choisi dans le groupe consistant en amidon hydroxyalkylé, amidon carboxyalkylé, ester d'amidon et des mélanges quelconques de ceux-ci, dans laquelle ledit dérivé d'amidon présente un poids moléculaire moyen Mw1 et un degré de substitution molaire MS1,
b) procurer un second dérivé d'amidon choisi dans le groupe consistant en amidon hydroxyalkylé, amidon carboxyalkylé, ester d'amidon et des mélanges quelconques de ceux-ci, ayant un poids moléculaire moyen Mw2 et un degré de substitution molaire MS2, où Mw1 est supérieur à Mw2 (Mw1 > Mw2), et MS2 est supérieur à MS1 (MS2 > MS1), dans laquelle le rapport pondéral du premier dérivé d'amidon au second dérivé d'amidon est de 1:9 à 9:1, et dans laquelle ledit second dérivé d'amidon présente un poids moléculaire moyen Mw2 de 30000 à 80000 daltons,
c) mélanger ledit premier dérivé d'amidon avec ledit second dérivé d'amidon, et
d) ajouter un ou plusieurs électrolytes.
